# EUROPÄISCHE PATENTSCHRIFT

(11) **EP 2 393 439 B1**
(45) Veröffentlichungstag und Bekanntmachung des Hinweises auf die Patenterteilung: **07.01.2015**
(21) Anmeldenummer: 09748107.1
(22) Anmeldetag: 03.11.2009
(51) Int. Cl.: A61B 17/30, A61B 18/14

(54) **CHIRURGISCHES INSTRUMENT**
SURGICAL INSTRUMENT
INSTRUMENT CHIRURGICAL

(30) Priorität: 03.02.2009 DE 102009007205
(43) Veröffentlichungstag der Anmeldung: 14.12.2011
(73) Patentinhaber: Aesculap AG, 78532 Tuttlingen (DE)
(72) Erfinder: KIENZLE, Karl-Ernst, 78532 Tuttlingen (DE); WEISSHAUPT, Dieter, 78194 Immendingen (DE)
(74) Vertreter: Hoeger, Stellrecht & Partner Patentanwälte
(86) Internationale Anmeldenummer: PCT/EP2009/064505
(87) Internationale Veröffentlichungsnummer: WO 2010/088974

(56) Entgegenhaltungen:
- EP-A1- 2 011 447
- WO-A1-2008/071750
- DE-U1-202007 016 233
- US-A- 4 452 106
- US-A- 5 334 215
- US-B2- 6 860 882

## Beschreibung

Die Erfindung betrifft ein chirurgisches Instrument mit zwei gegeneinander bewegbaren Branchen, an deren freiem Ende eine in einen Aufnahmeraum am distalen Ende der Branche eingeschobene Spitze auswechselbar gehalten ist, mit einer Fixiereinrichtung zur lösbaren Festlegung an der Branche, welche zwei elastisch auseinander biegbare, nebeneinander angeordnete Rastarme an einem der beiden Teile aufweist und einen sich quer zur Einschubrichtung der Spitze in die Branche erstreckenden Rastvorsprung am anderen der beiden Teile, der beim Einschieben der Spitze in den Aufnahmeraum der Branche zwischen die beiden Rastarme eingreift, diese elastisch auseinander biegt und am Ende der Einschubbewegung in einen Rücksprung an der Innenseite der Rastarme eintaucht, so dass sich die Rastarme nach dem elastischen Auseinanderbiegen wieder einander annähern.

Es sind chirurgische Instrumente dieser Art bekannt, die beispielsweise als Pinzette oder als Klemminstrument eingesetzt werden, und bei denen die Spitzen auswechselbar sind. Beispielsweise ist in der DE 20 2007 016 233 U1 eine chirurgische Pinzette beschrieben, bei der die in den Aufnahmeraum der Branche eingeschobene Spitze einmal durch spezielle Anlageflächen gegenüber der Branche in die richtige Winkelstellung verdreht wird und zum anderen durch eine getrennte Rastvorrichtung in axialer Richtung festgelegt wird. Der Aufbau ist jedoch relativ kompliziert, da getrennte Mittel zum Ausrichten der Spitze um die Längsachse der Branche einerseits und zum Festlegen der Spitze gegen eine Verschiebung in Längsrichtung der Branche andererseits notwendig sind.

In der US 6,860,882 B2 ist eine Pinzette beschrieben, bei der eine Spitze auswechselbar an einem Pinzettenkörper gehalten ist. Um das Ausfedern der Rastarme der Pinzettenspitze zu ermöglichen, muss der Aufnahmeraum des Pinzettenkörpers seitlich durchbrochen sein. Dadurch wird die Stabilität herabgesetzt, außerdem besteht in diesem Bereich der Durchbrechungen die Gefahr einer Verschmutzung und einer Verletzung.

Es ist Aufgabe der Erfindung, ein gattungsgemäßes chirurgisches Instrument in seinem Aufbau zu vereinfachen und insbesondere eine Verschmutzung und eine Verletzungsgefahr herabzusetzen.

Diese Aufgabe wird bei einem chirurgischen Instrument der eingangs beschriebenen Art erfindungsgemäß dadurch gelöst, dass sich der Aufnahmeraum von seinem Einsteckende in proximaler Richtung aufweitet.

Die Rastarme, die zwischen sich den Rastvorsprung aufnehmen, übernehmen dabei eine Doppelfunktion. Beim Eintreten des Rastvorsprungs zwischen die Rastarme werden diese elastisch aufgebogen und dadurch richten sich die beiden Rastarme relativ zu dem Rastvorsprung aus, das heißt die Winkelstellung der Spitze gegenüber der Branche wird dadurch festgelegt. Sobald der Rastvorsprung in den Rücksprung eingreift, erfolgt dadurch zusätzlich eine Festlegung der Spitze in axialer Richtung, das heißt in Einschubrichtung. Die beschriebene Ausgestaltung hat den Vorteil, dass der Benutzer die Spitze in den Aufnahmeraum einschieben kann, beim Einschieben wird die Spitze relativ zu dem Rastvorsprung durch eine Drehung um die Längsachse der Branche ausgerichtet und sobald der Rastvorsprung in den Rücksprung einrastet, erhält der Benutzer dadurch eine Rückmeldung, denn er spürt das Einschnappen des Rastvorsprunges in dem Rücksprung und die dadurch mögliche Rückbewegung der elastisch aufgebogenen Rastarme in die Ausgangsstellung.

Durch die Aufweitung des Aufnahmeraumes in proximaler Richtung kann der Aufnahmeraum seitlich geschlossen sein, trotzdem können die Rastarme des einen Teiles beim Zusammenschieben der beiden Teile ausfedern. Der geschlossene Aufnahmeraum stellt sicher, dass keine Verletzungen oder Verschmutzungen durch Wanddurchbrechungen im Bereich des Aufnahmeraumes auftreten.

Insbesondere kann die Innenwand des Aufnahmeraumes sich vom Einsteckende in proximaler Richtung konisch aufweiten.

Vorteilhaft ist es, wenn die Rastarme zwischen ihrem freien Ende und dem Rücksprung an ihrer Innenseite eine gegenüber der Einschubrichtung schräg verlaufende Aufgleitfläche aufweisen, so dass der gegenseitige Abstand der beiden einander zugewandten Aufgleitflächen in Richtung auf das freie Ende der Rastarme zunimmt. Durch diese Aufgleitflächen wird einmal das Einführen des Rastvorsprungs in den Zwischenraum zwischen den beiden Rastarmen erleichtert, zum anderen dienen diese Aufgleitflächen der Orientierung der Rastarme und damit der Spitze relativ zu der Branche, da sich der Rastvorsprung an die Aufgleitflächen anlegt und diese dadurch um die Längsachse der Spitze gegenüber der Branche so verdreht, dass die gewünschte Orientierung der Spitze relativ zur Branche eintritt.

Gemäß einer besonders bevorzugten Ausführungsform der Erfindung ist vorgesehen, dass der Abstand der dem freien Ende der Rastarme näher liegenden Übergänge des Rücksprunges in die Innenseite des Rastarmes größer ist als der Abstand der dem freien Ende abgewandten Übergänge des Rücksprunges in die Innenseite des Rastarmes. Mit anderen Worten ist die Stufe zwischen dem Rücksprung und der Aufgleitfläche für den Rastvorsprung niedriger auf der dem freien Ende zugewandten Seite des Rücksprunges als auf der dem freien Ende abgewandten Seite des Rücksprunges. Dadurch wird der Rastvorsprung beim Einschnappen in den Rücksprung gegen ein weiteres Vorschieben blockiert, das heißt der Rastvorsprung wirkt als Endanschlag für den Einschub der Spitze in den Aufnahmeraum, in umgekehrter Richtung kann aber der Rastvorsprung an dieser dem freien Ende zugewandten Stufe entlang gleiten, so dass beim Einschieben der Spitze der Rastvorsprung in den Rücksprung einschnappen kann und umgekehrt beim kräftigen Herausziehen der Spitze aus dem Aufnahmeraum der umgekehrte Vorgang eintritt.

Insbesondere kann der Rastvorsprung ein quer zur Einschubrichtung verlaufender Stift sein.

Es ist günstig, wenn der Rastvorsprung einen kreisförmigen Querschnitt aufweist. Der Rücksprung kann insbesondere den Querschnitt eines Kreissegmentes aufweisen.

In diesen Fällen ist es vorteilhaft, wenn der Radius des kreisförmigen Vorsprungs und des Kreissegments der Rücksprünge im Wesentlichen gleich ist, so dass der Rastvorsprung beim Eintauchen in die Rücksprünge flächig an diesen anliegt.

Während es grundsätzlich möglich ist, den Rastvorsprung an der Spitze und die Rastarme mit den Rücksprüngen an der Branche vorzusehen, wird eine Ausführungsform bevorzugt, bei der die Rastarme mit der Spitze fest verbunden sind und der Rastvorsprung fest mit der Branche verbunden ist.

Insbesondere können die Rastarme einstückig mit der Spitze oder mit einem Halter der Spitze ausgebildet sein und durch einen sich vom freien Ende der Rastarme ausgehend über die Rücksprünge hinaus erstreckenden Schlitz von einander getrennt sein. Dadurch wird die Herstellung erleichtert. Es genügt, die beiden Rastarme durch Einbringen des Schlitzes von einander zu trennen. Der Rastvorsprung kann insbesondere den Aufnahmeraum quer durchsetzen, es kann sich also beispielsweise um einen Stift handeln, der in entsprechenden Aufnahmeöffnungen der Wand des Aufnahmeraumes gelagert ist.

Vorteilhaft ist es, wenn der Aufnahmeraum an seinem Einsteckende am distalen Ende der Branche außenseitig dicht an der in den Aufnahmeraum eingeschobenen Spitze anliegt. Dadurch ergibt sich eine seitliche Führung der Spitze im Aufnahmeraum in diesem Bereich.

Bei einer bevorzugten Ausführungsform ist vorgesehen, dass der Spalt zwischen der in den Aufnahmeraum eingeschobenen Spitze und dem distalen Ende der Branche von einer Dichtmanschette überfangen ist.

Es ist dabei günstig, wenn die Dichtmanschette durch eine eine Fixiervertiefung und einen in die Fixiervertiefung eingreifenden Fixiervorsprung umfassende Rastverbindung in Einschubrichtung der Spitze gesichert ist. Beispielsweise kann die Fixiervertiefung eine Umfangsnut sein und der Fixiervorsprung eine umlaufende Ringschulter, die Fixiervertiefung kann sich sowohl in der Branche als auch in der Spitze befinden. Wenn sich die Fixiervertiefung in der Spitze befindet, ist es günstig, dass beim Auswechseln der Spitze die Dichtmanschette mit der Spitze von der Branche abgezogen und damit auch ausgewechselt wird.

Die nachfolgende Beschreibung bevorzugter Ausführungsformen der Erfindung dient im Zusammenhang mit der Zeichnung der näheren Erläuterung. Es zeigen:

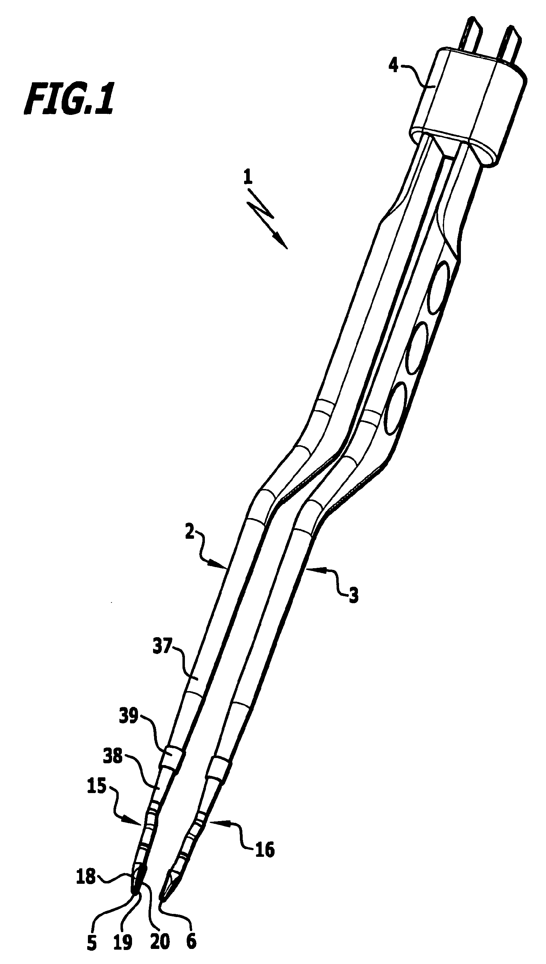

- Figur 1:: eine perspektivische Ansicht einer Pinzette mit zwei Branchen und auswechselbar daran gehaltenen Spitzen;

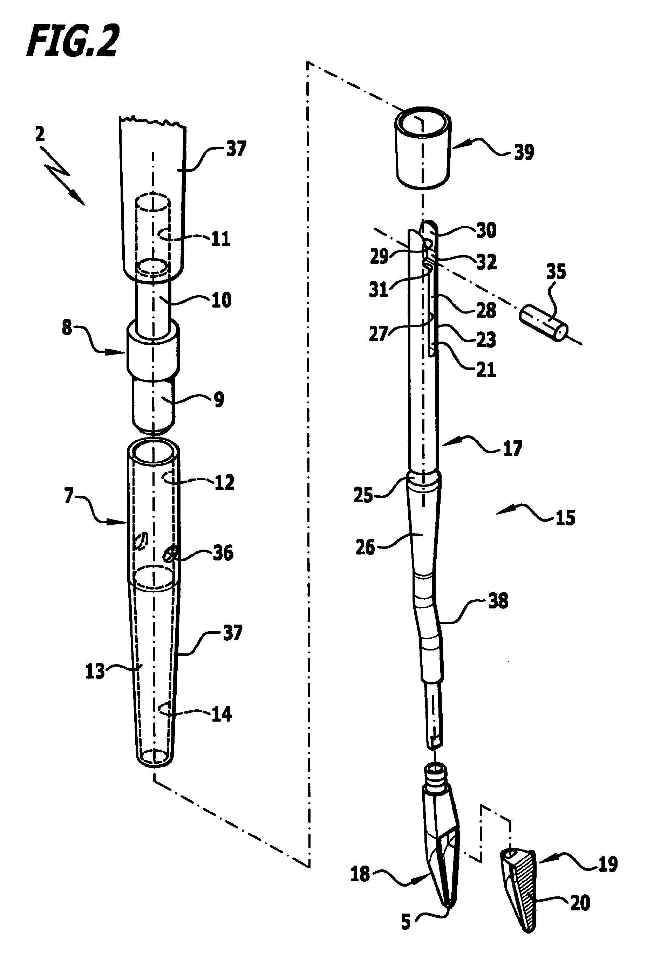

- Figur 2:: eine vergrößerte Detailansicht des distalen Endes einer Branche mit einem Aufnahmeraum und mit den Teilen der in den Aufnahmeraum einschiebbaren Spitze;

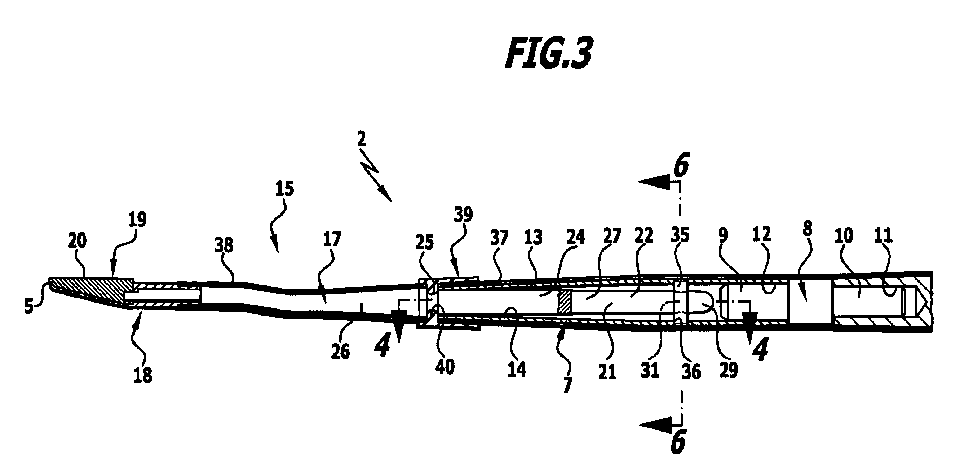

- Figur 3:: eine Längsschnittansicht des distalen Endbereiches einer Branche mit eingeschobener Spitze;

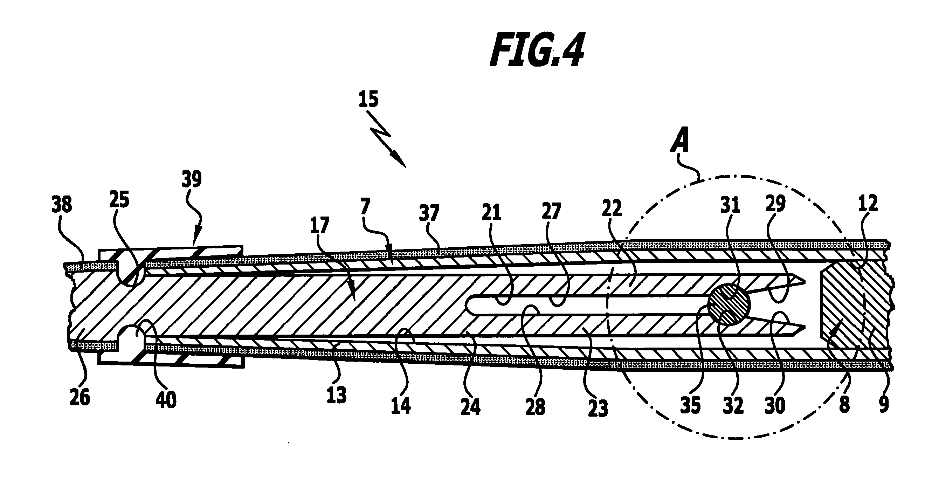

- Figur 4:: eine Schnittansicht längs Linie 4-4 in Figur 3;
- Figur 5:: eine vergrößerte Detailansicht des Bereiches A in Figur 4 und

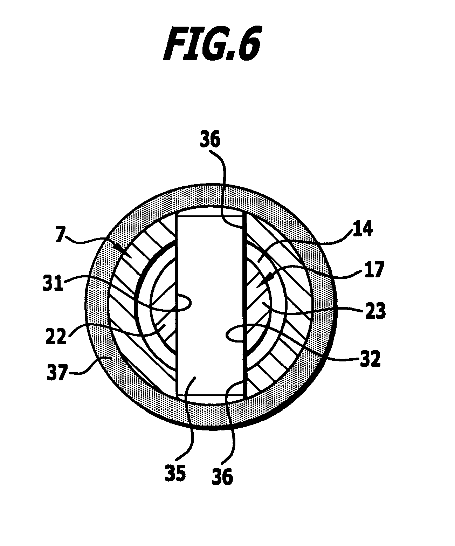

- Figur 6:: eine Schnittansicht längs Linie 6-6 in Figur 3.

Die Erfindung wird nachstehend am Beispiel einer Pinzette 1 erörtert, grundsätzlich kann die Erfindung jedoch an anderen chirurgischen Instrumenten Anwendung finden, bei denen an einer Branche eine auswechselbare Spitze gehalten ist, die durch Einschieben in einen Aufnahmeraum der Branche mit dieser verbunden werden kann, beispielsweise bei einem Klemmwerkzeug oder bei einem zangenähnlichen Instrument.

Die Pinzette 1 umfasst zwei nebeneinander angeordnete, längliche Branchen 2, 3, die an ihrem proximalen Ende über ein Verbindungselement 4 im Abstand zueinander festgelegt sind und die an ihrem distalen Ende jeweils ein Arbeitselement 5, 6 tragen. Die Branchen 2, 3 sind elastisch federnd gegen einander drückbar und können auch elastisch federnd auseinander gedrückt werden, so dass auch die beiden Arbeitselemente 5, 6 entsprechend gegen einander oder auseinander bewegt werden.

Die beiden Branchen 2, 3 sind in ihrem distalen Bereich im Wesentlichen gleich aufgebaut, nachstehend wird daher lediglich eine der beiden Branchen näher beschrieben. Am distalen Ende der Branche 2 weist diese eine hülsenförmige Verlängerung 7 auf, die über ein Zwischenstück 8 mit der jeweiligen Branche 2 verbunden ist. Das Zwischenstück 8 greift mit zapfenförmigen Abschnitten 9, 10 auf gegenüberliegenden Seiten in entsprechende zylindrische Bereiche 11 beziehungsweise 12 der Branche 2 beziehungsweise der Verlängerung 7 ein, die zapfenförmigen Abschnitte 9, 10 können in die zylindrischen Bereiche 11, 12 eingeschraubt sein oder in anderer Weise fest mit diesen verbunden sein, beispielsweise durch Verklebung.

Die hülsenförmige Verlängerung 7 ist in einem an das Zwischenstück 8 anschließenden proximalen Bereich zylindrisch ausgebildet. An diesen zylindrischen Bereich schließt sich ein in distaler Richtung einen abnehmenden Querschnitt aufweisender konischer Bereich 13 an. Die Verlängerung 7 umschließt damit einen Aufnahmeraum 14, der sich vom distalen Ende der Verlängerung 7 zunächst konisch erweitert und dann sich zylindrisch fortsetzt.

In diesen Aufnahmeraum 14 ist eine Spitze 15 oder 16 eingeschoben, die darin auswechselbar festgelegt ist.

Die Spitze 15 und in gleicher Weise die Spitze 16 ist aus verschiedenen Teilen aufgebaut, nämlich zunächst einem Halter 17 in Form eines länglichen Stabes und einem Maulteil 18, welches auf das distale Ende des Halters 17 aufgesteckt und dort mit diesem fest verbunden ist. In dem dargestellten Ausführungsbeispiel ist in das Maulteil 18 ein Klemmeinsatz 19 eingesetzt, der eine Klemmfläche 20 aufweist. Die Klemmflächen 20 der neben einander angeordneten Maulteile 18 der beiden Branchen 2, 3 sind dabei einander zugewandt.

An seinem proximalen Ende ist in den im Querschnitt kreiszylindrischen Halter 17 parallel zu dessen Längsachse verlaufend ein diametraler, durchgehender Schlitz 21 eingearbeitet, der den Halter 17 in zwei neben einander liegende Arme 22, 23 auftrennt. Die Länge der auf diese Weise entstehenden Arme 22, 23 ist bei dem dargestellten Ausführungsbeispiel etwa halb so groß wie die Länge eines zylindrischen, proximalen Abschnittes 24 des Halters 17, am distalen Ende dieses zylindrischen Abschnittes 24 schließt sich eine umlaufende Umfangsnut 25 an. Distal dieser Umfangsnut 25 folgt ein sich zum distalen Ende hin konisch verengender Abschnitt 26, dessen Konizität vorzugsweise der Konizität des konischen Bereiches 13 der Verlängerung 7 entspricht.

Ausgehend vom distalen Ende des Schlitzes 21 verlaufen die Innenseiten 27, 28 der beiden Arme 22, 23 zunächst parallel zueinander und gehen dann im Bereich des proximalen Endes der Arme 22, 23 in schräge Aufgleitflächen 29, 30 über, so dass sich die Breite des Schlitzes 21 zum proximalen Ende der Arme 22, 23 hin erweitert.

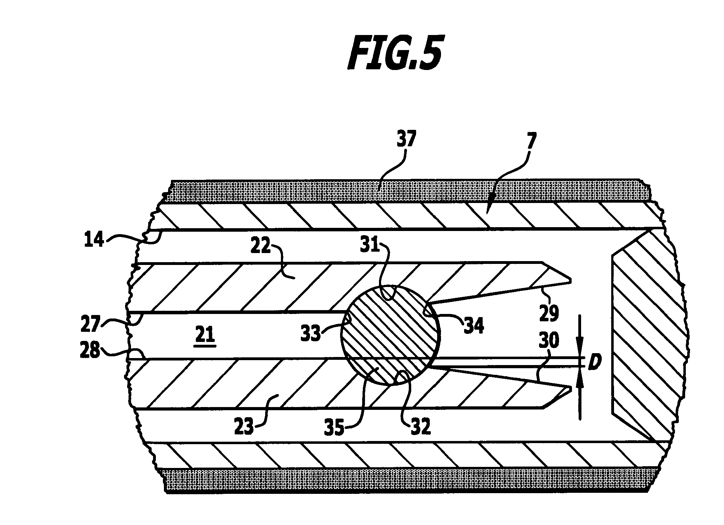

Im Übergangsbereich zwischen den parallel zueinander verlaufenden Bereichen der Innenseiten 27 und 28 und der sich daran anschließenden Aufgleitflächen 29, 30 ist in beiden Armen an deren Innenseite jeweils ein sich quer zur Längsrichtung der Arme 22, 23 erstreckender Rücksprung 31, 32 vorgesehen. Der Querschnitt dieser Rücksprünge 31, 32 ist kreissegmentförmig, das heißt der Boden der Rücksprünge 31, 32 folgt einem kreisbogenförmigen Querschnitt, der am distalen Ende des Rücksprunges 31, 32 über eine Kante 33 in die Innenseite 27 des Armes 22 übergeht, am proximalen Ende des Rücksprunges 31 dagegen über eine Kante 34 in die entsprechende sich anschließende Aufgleitfläche 29. Bei unverformten Armen 22, 23 liegen dadurch die Kanten 33 der beiden Branchen 2, 3 dichter bei einander als die proximalen Kanten 34, in Figur 5 ist die unterschiedliche Höhe der Kanten 33 und 34 durch die Differenz D gekennzeichnet.

Im zylindrischen Bereich der Verlängerung 7 durchsetzt ein im Querschnitt kreisförmiger Stift 35 den Aufnahmeraum 14, der Stift 35 ist dabei in seitlichen Öffnungen 36 in der Wand der Verlängerung 7 eingesetzt und dort in geeigneter Weise festgelegt, beispielsweise durch eine Verschweißung oder Verklebung. Der Außendurchmesser des Stiftes 35 entspricht im Wesentlichen dem Durchmesser der kreissegmentförmigen Rücksprünge 31, 32.

Um eine Spitze 15 mit einer Branche 2 zu verbinden, wird die Spitze 15 mit den proximalen Enden der Arme 22, 23 voran in den Aufnahmeraum 14 eingeschoben. Dabei legen sich die Aufgleitflächen 29, 30 an den den Aufnahmeraum 14 quer durchsetzenden Stift 35 an und drehen die Spitze 15 so, dass beide Aufgleitflächen 29, 30 flächig an dem Stift 35 anliegen, das heißt der Schlitz 21 wird in genau definierter Lage so orientiert, dass die Längsachse des Stiftes 35 in der Mittelebene des Schlitzes 21 verläuft. Durch die Anlage des Stiftes 35 an den Aufgleitflächen 29, 30 werden beim weiteren Vorschieben die Arme 22, 23 elastisch auseinander gebogen, bis der Stift 35 in die Rücksprünge 31, 32 einschnappt und in diesen aufgenommen wird. Die Arme 22, 23 federn dann in die Ausgangslage zurück und halten den Stift 35 innerhalb der beiden Rücksprünge 31, 32. Die Tatsache, dass die Kante 33 höher ausgebildet ist als die Kante 34 der Rücksprünge 31, 32 führt dazu, dass ein weiteres Einschieben der Spitze 15 verhindert wird. Umgekehrt ist es jedoch möglich, die Spitze, die normalerweise durch den Eingriff des Stiftes 35 in die Rücksprünge 31 und 32 in axialer Richtung und gegen eine Drehung gesichert in der Branche 2 gehalten ist, aus dieser auch wieder herausziehen. Dazu ist eine bestimmte Auszugskraft notwendig, die ausreicht, um den Stift 35 aus den Rücksprüngen 31, 32 austreten zu lassen, dabei werden die Arme 22 und 23 wieder elastisch aufgebogen. Da die Kante 34 niedriger ausgebildet ist als die Kante 33, ist ein solches Herausziehen möglich, allerdings muss dabei kräftig an der Spitze gezogen werden.

Die in den Aufnahmeraum 14 eingesetzte Spitze 15 wird in dem Aufnahmeraum einmal durch den Eingriff des Stiftes 35 in die Rücksprünge 31, 32 gehalten, zum anderen auch durch eine Anlage des zylindrischen Bereiches des Halters 17 an der Innenwand des konischen Bereiches 13 der Verlängerung 7, und zwar legt sich die Verlängerung 7 an ihrem distalen Ende an die Außenwand des Halters 17 an und führt diesen dadurch seitlich.

Die Branche 2 und daran anschließend die Verlängerung 7 sind bei dem dargestellten Ausführungsbeispiel von einem Isoliermantel 37 aus einem isolierenden Kunststoff umgeben, der sich bis an das distale Ende der Verlängerung 7 erstreckt und die Form eines Schrumpfschlauches haben kann.

In ähnlicher Weise ist auch der Halter von einem Isoliermantel 38 umgeben, dieser erstreckt sich in proximaler Richtung bis zu der Umfangsnut 25.

Der Spalt zwischen dem distalen Ende des Isoliermantels 37 und dem proximalen Ende des Isoliermantels 38 wird von einer ringförmigen Dichtmanschette 39 überfangen, die an ihrer Innenseite eine wulstförmige, umlaufende Ringschulter 40 trägt. Diese Ringschulter 40 greift in die Umfangsnut 25 ein und sichert dadurch die Dichtmanschette 39 gegen eine axiale Verschiebung, beim Herausziehen der Spitze 15 aus der Branche 2 kann dadurch auch die Dichtmanschette 39 mit abgezogen werden. Das heißt beim Auswechseln einer Spitze wird auch die Dichtmanschette mit ausgewechselt.

## Patentansprüche

1. Chirurgisches Instrument mit zwei gegeneinander bewegbaren Branchen (2, 3), an deren freiem Ende eine in einen Aufnahmeraum (14) am distalen Ende der Branche (2, 3) eingeschobene Spitze (15, 16) auswechselbar gehalten ist, mit einer Fixiereinrichtung zur lösbaren Festlegung der Spitze an der Branche (2, 3), welche zwei elastisch auseinander biegbare, nebeneinander angeordnete Rastarme (22, 23) an einem der beiden Teile (17) aufweist und einen sich quer zur Einschubrichtung der Spitze (15, 16) in die Branche (2, 3) erstreckenden Rastvorsprung (35) am anderen der beiden Teile (2, 3), der beim Einschieben der Spitze (15, 16) in den Aufnahmeraum (14) der Branche (2, 3) zwischen die beiden Rastarme (22, 23) eingreift, diese elastisch auseinander biegt und am Ende der Einschubbewegung in einen Rücksprung (31, 32) an der Innenseite (27, 28) der Rastarme (22, 23) eintaucht, so dass sich die Rastarme (22, 23) nach dem elastischen Auseinanderbiegen wieder einander annähern, **dadurch gekennzeichnet, dass** sich der Aufnahmeraum (14) von seinem Einsteckende in proximaler Richtung aufweitet.

2. Chirurgisches Instrument nach Anspruch 1, **dadurch gekennzeichnet, dass** die Innenwand des Aufnahmeraums (14) sich vom Einsteckende in proximaler Richtung konisch aufweitet.

3. Chirurgisches Instrument nach Anspruch 1 oder 2, **dadurch gekennzeichnet, dass** die Rastarme (22, 23) zwischen ihrem freien Ende und dem Rücksprung (31, 32) an ihrer Innenseite eine gegenüber der Einschubrichtung schräg verlaufende Aufgleitfläche (29, 30) aufweisen, so dass der gegenseitige Abstand der beiden einander zugewandten Aufgleitflächen (29, 30) in Richtung auf das freie Ende der Rastarme (22, 23) zunimmt.

4. Chirurgisches Instrument nach einem der voranstehenden Ansprüche, **dadurch gekennzeichnet, dass** der Abstand der dem freien Ende der Rastarme (22, 23) näher liegenden Übergänge (34) des Rücksprunges (31, 32) in die Innenseite (27, 28) des Rastarmes (22, 23) größer ist als der Abstand der dem freien Ende abgewandten Übergänge (33) des Rücksprunges (31, 32) in die Innenseite des Rastarmes (22, 23).

5. Chirurgisches Instrument nach einem der voranstehenden Ansprüche, **dadurch gekennzeichnet, dass** der Rastvorsprung (35) ein quer zur Einschubrichtung verlaufender Stift ist.

6. Chirurgisches Instrument nach einem der voranstehenden Ansprüche, **dadurch gekennzeichnet, dass** der Rastvorsprung (35) einen kreisförmigen Querschnitt aufweist.

7. Chirurgisches Instrument nach einem der voranstehenden Ansprüche, **dadurch gekennzeichnet, dass** der Rücksprung (31, 32) den Querschnitt eines Kreissegmentes aufweist.

8. Chirurgisches Instrument nach den Ansprüchen 6 und 7, **dadurch gekennzeichnet, dass** der Radius des kreisförmigen Rastvorsprunges (35) und des Kreissegmentes der Rücksprünge (31, 32) im Wesentlichen gleich ist.

9. Chirurgisches Instrument nach einem der voranstehenden Ansprüche, **dadurch gekennzeichnet, dass** die Rastarme (22, 23) mit der Spitze (15, 16) fest verbunden sind und der Rastvorsprung (35) fest mit der Branche (2, 3) verbunden ist.

10. Chirurgisches Instrument nach Anspruch 9, **dadurch gekennzeichnet, dass** die Rastarme (22, 23) einstückig mit der Spitze (15, 16) oder mit einem Halter (17) der Spitze (15, 16) ausgebildet sind und durch einen sich vom freien Ende der Rastarme (22, 23) ausgehend über die Rücksprünge (31, 32) hinaus erstreckenden Schlitz (21) von einander getrennt sind.

11. Chirurgisches Instrument nach einem der Ansprüche 9 oder 10, **dadurch gekennzeichnet, dass** der Rastvorsprung (35) den Aufnahmeraum (14) quer durchsetzt.

12. Chirurgisches Instrument nach einem der voranstehenden Ansprüche, **dadurch gekennzeichnet, dass** der Aufnahmeraum (14) an seinem Einsteckende am distalen Ende der Branche (2, 3) außenseitig dicht an der in den Aufnahmeraum (14) eingeschobenen Spitze (15, 16) anliegt.

13. Chirurgisches Instrument nach einem der voranstehenden Ansprüche, **dadurch gekennzeichnet, dass** der Spalt zwischen der in den Aufnahmeraum (14) eingeschobenen Spitze (15, 16) und dem distalen Ende der Branche (2, 3) von einer Dichtmanschette (39) überfangen ist.

14. Chirurgisches Instrument nach Anspruch 13, **dadurch gekennzeichnet, dass** die Dichtmanschette (39) durch eine eine Fixiervertiefung (25) und einen in die Fixiervertiefung (25) eingreifenden Fixiervorsprung (40) umfassende Rastverbindung in Einschubrichtung der Spitze (15, 16) gesichert ist.

## Claims

1. Surgical instrument with two legs (2, 3) movable relative to each other, a tip (15, 16) inserted in a receiving space (14) at the distal end of the leg (2, 3) being exchangeably held at the free end of the legs, with a fixing device for releasable fixation of the tip to the leg (2, 3), the fixing device comprising on one of the two parts (17) two locking arms (22, 23) which are arranged next to each other and are elastically bendable apart, and comprising on the other one of the two parts (2, 3) a locking projection (35) extending transversely to the direction of insertion of the tip (15, 16) into the leg (2, 3), the locking projection, when inserting the tip (15, 16) into the receiving space (14) of the leg (2, 3), engaging between the two locking arms (22, 23), bending these elastically apart, and, at the end of the inserting movement, entering a recess (31, 32) on the inner side (27, 28) of the locking arms (22, 23), so that the locking arms (22, 23) approach each other again after they have been elastically bent apart, **characterized in that** the receiving space (14) widens in the proximal direction from its insertion end.

2. Surgical instrument in accordance with claim 1, **characterized in that** the inner wall of the receiving space (14) widens conically from the insertion end in the proximal direction.

3. Surgical instrument in accordance with claim 1 or 2, **characterized in that** the locking arms (22, 23) have on their inner side between their free end and the recess (31, 32) a slide surface (29, 30) extending obliquely in relation to the direction of insertion, so that the mutual spacing between the two slide surfaces (29, 30) facing each other increases in the direction towards the free end of the locking arms (22, 23).

4. Surgical instrument in accordance with any one of the preceding claims, **characterized in that** the spacing between the continuations (34) of the recess (31, 32) that are closer to the free end of the locking arms (22, 23) into the inner side (27, 28) of the locking arm (22, 23) is greater than the spacing between the continuations (33) of the recess (31, 32) that are remote from the free end into the inner side of the locking arm (22, 23).

5. Surgical instrument in accordance with any one of the preceding claims, **characterized in that** the locking projection (35) is a pin extending transversely to the direction of insertion.

6. Surgical instrument in accordance with any one of the preceding claims, **characterized in that** the locking projection (35) has a circular cross section.

7. Surgical instrument in accordance with any one of the preceding claims, **characterized in that** the recess (31, 32) has the cross section of a segment of a circle.

8. Surgical instrument in accordance with claims 6 and 7, **characterized in that** the radius of the circular locking projection (35) and of the circular segment of the recesses (31, 32) is substantially identical.

9. Surgical instrument in accordance with any one of the preceding claims, **characterized in that** the locking arms (22, 23) are firmly connected to the tip (15, 16), and the locking projection (35) is firmly connected to the leg (2, 3).

10. Surgical instrument in accordance with claim 9, **characterized in that** the locking arms (22, 23) are formed integrally with the tip (15, 16) or with a holder (17) of the tip (15, 16) and are separated from each other by a slot (21) extending from the free end of the locking arms (22, 23) beyond the recesses (31, 32).

11. Surgical instrument in accordance with claim 9 or 10, **characterized in that** the locking projection (35) extends transversely through the receiving space (14).

12. Surgical instrument in accordance with any one of the preceding claims, **characterized in that** the receiving space (14), at its insertion end at the distal end of the leg (2, 3), bears tightly against the outside of the tip (15, 16) inserted in the receiving space (14).

13. Surgical instrument in accordance with any one of the preceding claims, **characterized in that** the gap between the tip (15, 16) inserted in the receiving space (14) and the distal end of the leg (2, 3) is covered by a sealing collar (39).

14. Surgical instrument in accordance with claim 13, **characterized in that** the sealing collar (39) is secured in the direction of insertion of the tip (15, 16) by a locking connection comprising a fixing recess (25) and a fixing projection (40) engaging in the fixing recess (25).

## Revendications

1. Instrument chirurgical, comportant deux branches (2, 3), qui peuvent être déplacées l'une contre l'autre et sur l'extrémité libre desquelles est maintenue de manière interchangeable une pointe (15, 16) insérée dans un logement (14) au niveau de l'extrémité distale de la branche (2, 3), comportant un dispositif de fixation pour la fixation amovible de la pointe sur la branche (2, 3), lequel comporte, au niveau de l'une des deux parties (17), deux bras de blocage (22, 23), disposés l'un à côté de l'autre et élastiquement flexibles pour s'écarter l'un de l'autre, et, sur l'autre des deux parties (2, 3), une saillie de blocage (35), qui est orientée transversalement au sens d'introduction de la pointe (15, 16) dans la branche (2, 3) et qui, au moment de l'introduction de la pointe (15, 16) dans le logement (14) de la branche (2, 3), s'engage entre les deux bras de blocage (22, 23), les fléchit élastiquement pour les écarter l'un de l'autre et, à la fin du mouvement d'introduction, s'engage dans un retrait (31, 32) sur la face intérieure (27, 28) des bras de blocage (22, 23), de telle sorte que lesdits bras de blocage (22, 23) se rapprochent à nouveau après leur mouvement de flexion élastique pour s'écarter l'un de l'autre, **caractérisé en ce que** le logement (14) s'élargit depuis son extrémité d'introduction dans la direction proximale.

2. Instrument chirurgical selon la revendication 1, **caractérisé en ce que** la paroi intérieure du logement (14) s'élargit en forme de cône depuis son extrémité d'introduction dans la direction proximale.

3. Instrument chirurgical selon la revendication 1 ou 2, **caractérisé en ce que** les bras de blocage (22, 23) comportent, entre leur extrémité libre et le retrait (31, 32) sur leur face intérieure, une surface de glissement (29, 30) s'étendant en oblique par rapport au sens d'introduction, de telle sorte que la distance réciproque entre les deux surfaces de glissement (29, 30) orientées l'une vers l'autre augmente dans la direction vers l'extrémité libre des bras de blocage (22, 23).

4. Instrument chirurgical selon l'une quelconque des revendications précédentes, **caractérisé en ce que** la distance entre les zones de transition (34), plus proches de l'extrémité libre des bras de blocage (22, 23), du retrait (31, 32) dans la face intérieure (27, 28) du bras de blocage (22, 23) est supérieure à la distance entre les zones de transition (33), opposées à l'extrémité libre, du retrait (31, 32) dans la face intérieure du bras de blocage (22, 23).

5. Instrument chirurgical selon l'une quelconque des revendications précédentes, **caractérisé en ce que** la saillie de blocage (35) est un tenon orienté transversalement au sens d'introduction.

6. Instrument chirurgical selon l'une quelconque des revendications précédentes, **caractérisé en ce que** la saillie de blocage (35) possède une section transversale circulaire.

7. Instrument chirurgical selon l'une quelconque des revendications précédentes, **caractérisé en ce que** le retrait (31, 32) a la section transversale d'un segment de cercle.

8. Instrument chirurgical selon les revendications 6 et 7, **caractérisé en ce que** le rayon de la saillie de blocage (35) circulaire et le rayon du segment de cercle des retraits (31, 32) sont sensiblement identiques.

9. Instrument chirurgical selon l'une quelconque des revendications précédentes, **caractérisé en ce que** les bras de blocage (22, 23) sont reliés de manière fixe à la pointe (15, 16) et la saillie de blocage (35) est reliée de manière fixe à la branche (2, 3).

10. Instrument chirurgical selon la revendication 9, **caractérisé en ce que** les bras de blocage (22, 23) sont réalisés d'un seul tenant avec la pointe (15, 16) ou avec un support (17) de la pointe (15, 16) et sont séparés l'un de l'autre par une fente (21) qui s'étend depuis l'extrémité libre des bras de blocage (22, 23) au-delà des retraits (31, 32).

11. Instrument chirurgical selon la revendication 9 ou 10, **caractérisé en ce que** la saillie de blocage (35) traverse transversalement le logement (14).

12. Instrument chirurgical selon l'une quelconque des revendications précédentes, **caractérisé en ce que** le logement (14), à son extrémité d'introduction au niveau de l'extrémité distale de la branche (2, 3), est en appui étanche, à l'extérieur, contre la pointe (15, 16) insérée dans le logement (14).

13. Instrument chirurgical selon l'une quelconque des revendications précédentes, **caractérisé en ce que** la fente entre la pointe (15, 16), insérée dans le logement (14), et l'extrémité distale de la branche (2, 3) est recouverte par une manchette d'étanchéité (39).

14. Instrument chirurgical selon la revendication 13, **caractérisé en ce que** la manchette d'étanchéité (39) est immobilisée dans le sens d'introduction de la pointe (15, 16) par un assemblage par blocage comportant une profondeur de fixation (25) et une saillie de fixation (40) s'engageant dans la profondeur de fixation (25).
